# EUROPÄISCHE PATENTSCHRIFT

(11) **EP 2 259 811 B1**
(45) Veröffentlichungstag und Bekanntmachung des Hinweises auf die Patenterteilung: **03.04.2019**
(21) Anmeldenummer: 09711811.1
(22) Anmeldetag: 20.02.2009
(51) Int. Cl.: A61M 1/34, A61M 1/36

(54) **BLUTVOLUMENMONITOR UND BLUTBEHANDLUNGSVORRICHTUNG**
BLOOD VOLUME MONITOR AND BLOOD TREATMENT DEVICE
MONITEUR DU VOLUME SANGUIN ET DISPOSITIF DE TRAITEMENT DU SANG

(30) Priorität: 22.02.2008 DE 102008010531
(43) Veröffentlichungstag der Anmeldung: 15.12.2010
(73) Patentinhaber: Fresenius Medical Care Deutschland GmbH, 61352 Bad Homburg (DE)
(72) Erfinder: MOISSL, Ulrich, 61184 Karben (DE); CHAMNEY, Paul, Hertfordshire HP23 4EW (GB); WABEL, Peter, 64287 Darmstadt (DE)
(74) Vertreter: Bobbert & Partner Patentanwälte PartmbB
(86) Internationale Anmeldenummer: PCT/EP2009/001228
(87) Internationale Veröffentlichungsnummer: WO 2009/103550

(56) Entgegenhaltungen:
- EP-A- 1 226 838
- EP-A- 1 287 838
- EP-A- 1 900 384
- WO-A-93/00938
- WO-A-2004/004804

## Beschreibung

Die vorliegende Erfindung betrifft einen Blutvolumenmonitor, konfiguriert zum Ausführen eines Verfahrens zum Betreiben einer Blutbehandlungsvorrichtung für die extrakorporale Blutbehandlung eines Patienten gemäß dem Oberbegriff des Anspruchs 1. Sie betrifft ferner eine Blutbehandlungsvorrichtung gemäß dem Oberbegriff des Anspruchs 15.

Zum Entfernen harnpflichtiger Substanzen aus dem Blut dialysepflichtiger Patienten werden Verfahren zur Blutreinigung bzw. Blutbehandlung wie die Hämodialyse, die Hämofiltration oder die Hämodiafiltration eingesetzt.

Der dabei regelmäßig ebenfalls erforderliche Entzug von überschüssigem Körperwasser erfolgt mittels Ultrafiltration. Diese führt regelmäßig zu einer Reduktion des Blutvolumens des Patienten. Dabei kann eine zu starke oder zu schnelle Reduktion zu hypotonen Episoden des Patienten führen. Die Toleranz der Patienten gegen Volumenreduktion ist hierbei individuell sehr unterschiedlich; bei kritischen Patientenkollektiven, z. B. Diabetikern, Atherosklerosepatienten, treten unerwünschte Episoden deutlich gehäuft auf. Der Vorgang der Ultrafiltration und somit auch die Blutvolumenregelung ist daher sicherheitskritisch.

Zur Vermeidung solcher Zustände sind aus der Praxis Vorrichtungen wie bspw. der von der Fa. Fresenius Medical Care vertriebene Blutvolumenmonitor (BVM) bekannt. Dieser Monitor ist als Zusatzgerät für Dialysevorrichtungen erhältlich und kann als Einschubmodell in diese integriert werden. Der Monitor misst das Blutvolumen (BV) und gibt das Messergebnis als sogenanntes BVM-Signal aus. Dieses Signal kann ein relatives Blutvolumen (RBV) im Vergleich zum Blutvolumen zu Beginn der Behandlung angeben.

Manche Blutvolumenmonitore erlauben eine automatische Rückkopplung zwischen dem BVM-Signal und der Ultrafiltrationssteuerung der Dialysevorrichtung. Hierdurch lassen sich hypotensive Episoden wirkungsvoll vermeiden, indem die Ultrafiltration rechtzeitig vermindert oder gar unterbrochen wird, wenn sich der gemessene Blutvolumenwert bestimmten Grenzwerten annähert. Ein Abfall des Blutvolumens des behandelten Patienten auf ein für diesen kritisches Blutvolumen aufgrund eines zu ausgeprägten Flüssigkeitsentzugs durch die Ultrafiltration wird somit gar nicht erst erreicht.

Im Zusammenhang mit der Rückkopplung zwischen dem BVM-Signal und der Ultrafiltrationssteuerung der Dialysevorrichtung kann es jedoch vorkommen, dass die Ultrafiltrationssteuerung basierend auf dem BVM-Signal verringert oder gar gestoppt wird, obwohl sich der Patient nicht in einem kritischen Kreislaufzustand befindet und auch nicht auf einen solchen zusteuert. Da der Patient in solchen Fällen kreislaufstabil ist und auch ohne Veränderung der Ultrafiltration bzw. der Ultrafiltrationsrate kreislaufstabil bleibt, gibt das BVM-Signal in diesen Fällen ein (Mess-) Artefakt wieder.

Aus der WO 2004/004804 A1 ist ein Verfahren und eine Vorrichtung zur Bestimmung des Blutvolumens während einer extrakorporalen Blutbehandlung bekannt.

Aufgabe der vorliegenden Erfindung ist es, einen verbesserten Blutvolumenmonitor, konfiguriert zum Ausführen eines Verfahrens zum Betreiben einer Blutbehandlungsvorrichtung für die extrakorporale Blutbehandlung eines Patienten anzugeben.

Ein weiteres Ziel der vorliegenden Erfindung besteht darin, eine verbesserte Blutbehandlungsvorrichtung vorzuschlagen.

Die vorliegende Erfindung wird gelöst durch den Blutvolumenmonitor, konfiguriert zum Ausführen eines Verfahrens mit den Merkmalen des Anspruchs 1. Sie wird ferner gelöst durch eine Blutbehandlungsvorrichtung mit den Merkmalen des Anspruchs 15.

Erfindungsgemäß wird somit ein Blutvolumenmonitor, konfiguriert zum Ausführen eines Verfahrens zum Betreiben einer Blutbehandlungsvorrichtung, insbesondere einer Dialysevorrichtung, für die extrakorporale Behandlung des Blutes eines Patienten vorgeschlagen, bei dessen Ausführung wenigstens ein Wert das Blutvolumen oder dessen Entwicklung (d. h. Veränderung über die Zeit, wie unten stehend näher ausgeführt wird) betreffend gewonnen wird.

Unter dem Begriff *"Wert das Blutvolumen betreffend"* wird erfindungsgemäß jeder dem Fachmann als solcher bekannter Wert verstanden, mittels welchem Rückschlüsse auf die Kreislaufstabilität des Patienten möglich sind. Dieser Wert kann das absolute Blutvolumen des Patienten beziffern. Der Wert kann jedoch auch ein relatives Blutvolumen (RBV) angeben, welches sich auf ein weiteres Blutvolumen, z. B ein Ausgangsvolumen zu Beginn der Behandlung bezieht. Weitere Werte wie der Hämatokrit u. a. sind ebenfalls von der Erfindung umfasst, sofern sie einen Rückschluss auf das Blutvolumen erlauben. Andere Werte betreffen die Blutdichte, den Blutwassergehalt, die Hämoglobinkonzentration, aber auch unmittelbare Messwerte wie die optische Dichte oder die Ultraschalllaufzeit bzw. -geschwindigkeit und dergleichen.

Unter dem Begriff *"Gewinnen des Wertes"* wird erfindungsgemäß jeder Vorgang verstanden, welcher einen Rückschluss auf die Höhe des Wertes oder dessen Zugehörigkeit zu einem Wertebereich ermöglicht oder den Wert auf eine andere Weise charakterisiert. Unter *"Gewinnen des Wertes"* wird insbesondere das Messen dieses Wertes verstanden, wobei sowohl eine direkte als auch eine indirekte Messung von der Erfindung umfasst ist. Zu *"Gewinnen"* zählt jedoch auch ein Errechnen des Wertes aus anderen bekannten Größen sowie jedes anderweitige Rückschließen auf den Wert.

Der Wert des Blutvolumens wird gemäß dem Verfahren zu wenigstens einem Zeitpunkt der Blutbehandlung gewonnen, wobei es sich bei diesem Zeitpunkt um einen solchen vor (insbesondere unmittelbar vor) oder während der Blutbehandlung handeln kann. Dabei ist das Gewinnen des jeweils aktuellen Wertes des Blutvolumens zu einer Reihe von Zeitpunkten möglich. Diese Zeitpunkte können vorgegebene zeitliche Abstände zueinander haben. Sie können jedoch im Verlauf der Blutbehandlung zeitlich variabel angepasst werden. Die Abstände können stets gleich oder aber unterschiedlich sein. Der Wert kann auch kontinuierlich gewonnen werden.

Unter einem *"Signal"* wird erfindungsgemäß ein kontinuierliches oder ein diskontinuierliches, nur an eine Einrichtung der Blutbehandlungsvorrichtung oder an mehrere Einrichtungen der Blutbehandlungsvorrichtung gerichtetes Signal verstanden. Ein Signal kann erfindungsgemäß somit eine an eine erste Einrichtung und zugleich eine an eine zweite Einrichtung übermittelte Information umfassen. Die Arten der Informationen können dabei gleich oder unterschiedlich sein. So kann eine Information analog, die andere digital sein. Eine Information kann elektrischer Art sein, die andere optischer Art, usw.

Das Verfahren umfasst ferner ein Gewinnen einer Aussage über einen in einem Abschnitt der Blutbehandlungsvorrichtung oder in einem extrakorporalen Kreislauf herrschenden Druck, insbesondere Blutdruck, oder dessen Entwicklung. Der Begriff *"Gewinnen"* wird erfindungsgemäß wie oben erläutert verstanden.

Unter der Aussage *"den in einem extrakorporalen Kreislauf herrschenden Druck betreffend"* wird erfindungsgemäß sowohl ein absoluter Wert, eine Wertveränderung, ein relativer Wert sowie jede andere Aussage verstanden, mittels welcher der Fachmann relevante Druckereignisse und insbesondere Blutdruckereignisse in einem Abschnitt der Blutbehandlungsvorrichtung oder im extrakorporalen Kreislauf beurteilen kann. Die Aussage kann die (Blut-)Druckhöhe sein. Unter *Aussage"* zählen jedoch auch technische Daten des extrakorporalen Kreislaufs wie eine Durchflussgeschwindigkeit durch Schlauchabschnitte, eine Pumpendrehzahl und dergleichen.

Das Verfahren umfasst ferner ein Überprüfen auf ein Vorliegen einer Koinzidenz zwischen dem Blutvolumen -oder wenigstens einem Wert das Blutvolumen zu wenigstens einem Zeitpunkt oder dessen Entwicklung über wenigstens zwei Zeitpunkte der Blutbehandlung betreffend - und dem Blutdruck - oder einer Aussage über einen in einem Abschnitt der Blutbehandlungsvorrichtung oder in einem extrakorporalen Kreislauf herrschenden Druck, insbesondere Blutdruck - oder jeweils deren Entwicklungen.

Unter einem *"Überprüfen auf ein Vorliegen einer Koinzidenz"* wird erfindungsgemäß ein Analysieren, ein Untersuchen, ein Beobachten oder dergleichen verstanden, jeweils mit dem Ziel, eine gegebenenfalls vorhandene Koinzidenz im Sinne der vorliegenden Erfindung zu erkennen.

Unter einem Überprüfen kann erfindungsgemäß ganz allgemein eine zielgerichtete Beobachtung oder Informationserhebung verstanden werden. Vorzugsweise dient ein Überprüfen dem Erkennen eines Zusammenhangszwischen Ereignissen oder Verläufen.

Zum Zwecke des Überprüfens im Sinne der vorliegenden Erfindung werden vorzugsweise mathematische Modelle oder Algorithmen, insbesondere Schwellenwertmodelle, Trendanalysen oder dergleichen verwendet.

Der Begriff der *"Koinzidenz"* wie hierin verwendet umfasst - ohne jeweils hierauf beschränkt zu sein - ein zeitgleiches Auftreten von zwei Ereignissen.

Der Begriff der Koinzidenz umfasst ferner einen ursächlichen Zusammenhang, gleich ob sich dieser zeitgleich oder zeitversetzt zeigt. Koinzidenz umfasst ferner sowohl eine positive Korrelation als auch eine negative Korrelation.

Mit dem Einsatz des Verfahrens erzielbare Vorteile umfassen beispielsweise die Möglichkeit, aufgrund der Überprüfung des Vorliegens von Koinzidenzen Artefakte bei der Blutvolumenmessung zu erkennen. Liegen Artefakte vor, so können nicht erforderliche, in der Praxis in Unkenntnis des Vorliegens eines Artefakts aber vorgenommene Eingriffe in die Blutbehandlung - wie ein Verringern einer Filtrationsleistung, ein Ausgeben eines Alarms oder dergleichen - ohne negative Folgen, insbesondere für den Patienten, unterbleiben. Zudem kann das Erkennen einer Koinzidenz das Ausführen von korrigierenden Verfahrensschritten veranlassen.

Vorteilhafte Weiterbildungen der Erfindung sind jeweils Gegenstand der Unteransprüche.

So umfasst das Verfahren in einer bevorzugten Ausführungsform ferner das Ausgeben wenigstens eines ersten Signals zum Steuern oder Regeln der Blutbehandlungsvorrichtung und insbesondere zum Steuern oder Regeln einer Ultrafiltration oder einer Ultrafiltrationsrate. Bei der Ausgabe des Signals wird der gewonnene Blutvolumenwert berücksichtigt. Im Rahmen der vorliegenden Erfindung wird hierunter verstanden, dass beispielsweise ein an die Blutbehandlungsvorrichtung zu einem Zeitpunkt eines niedrigen Blutvolumens abgegebenes Steuersignal die Blutbehandlungsvorrichtung zu einer Verminderung der Ultrafiltrationsleistung bzw. - rate anregt, gemessen an einer Ultrafiltrationsleistung bzw. -rate, die zu einem Zeitpunkt ausgegeben wird, zu welchem ein höherer Blutvolumenwert gewonnen wird. In diesem Zusammenhang sei darauf hingewiesen, dass unter *"Ausgeben"* des Signals bereits auch ein *"Erzeugen"* - also ein Ermitteln, ein Berechnen oder dergleichen - des Signals zu verstehen ist.

Das Verfahren umfasst in dieser Ausführungsform ferner ein Anpassen des ersten Signals, welches zum Steuern oder Regeln der Blutbehandlungsvorrichtung und insbesondere der Ultrafiltrationsrate abgegeben wird, wobei bei diesem Signal die gewonnene Aussage über den herrschenden Druck und insbesondere Blutdruck berücksichtigt wird.

Die Berücksichtigung der gewonnenen Aussage über den Druck bzw. Blutdruck führt zu einem ersten Signal, welches die Blutbehandlungsvorrichtung derart steuert oder regelt, dass die Ultrafiltrationsleistung und/ oder -rate beispielsweise zurückgefahren bzw. vermindert wird, so dass einem Erreichen kreislaufkritischer Zustände des Patienten wirkungsvoll vorgebeugt wird.

Das Anpassen des ersten Signals kann hierbei auch derart erfolgen, dass neben der Abgabe einer ersten Information auch eine zweite Information zur Verwendung durch die Blutbehandlungsvorrichtung ausgegeben wird, wobei die zweite Information die Verringerung der Ultrafiltrationsleistung und/ oder -rate bewirkt. So kann auch ein kritisches RBV-Signal unverändert abgegeben werden, wobei die Ultrafiltration aber durch ein zweites Signal, welches z. B. die Filtrationspumpe anhält und bei kritischen RBV-Signalen zusätzlich ausgegeben wird, gestoppt werden kann.

Es wird darauf hingewiesen, dass im Zusammenhang mit der vorliegenden Erfindung der Begriff des *"Steuerns"* vom Fachmann stets dann auch durch ein *"Regeln"* zu ersetzen ist und letzeres ebenfalls von der vorliegenden Erfindung umfasst ist, wenn dies für den Fachmann anhand der vorliegenden Unterlagen erkennbar und aufgrund seines Fachwissens ausführbar ist. Ferner gilt im Zusammenhang mit der vorliegenden Erfindung, dass jedes Mal dann, wenn von der Ultrafiltration oder Ultrafiltrationsleistung und ihrer Steuerung oder Regelung die Rede ist, auch die Ultrafiltrationsrate zu verstehen ist - wobei dies auch umgekehrt gilt -, sofern der Fachmann die Anwendbarkeit der Aussage auf den jeweils anderen Begriff erkennt.

In einer weiter bevorzugten Ausführungsform wird ein Verfahren vorgeschlagen, welches ein Verändern der Höhe des ausgegebenen ersten Signals zum Steuern der Blutbehandlungsvorrichtung unter Berücksichtigung der gewonnenen Aussage, insbesondere durch Addition eines druckproportionalen Wertes zum Signal, umfasst. Die Höhe des Signals kann dabei jedoch auch vermindert werden. Dabei kann das Signal bis auf eine Höhe vermindert werden, in welcher die Blutbehandlungseinrichtung seine Wirkung einstellt und bspw. eine Filterung unterbleibt.

Das Verfahren weist in einer weiteren erfindungsgemäßen Ausführungsform das Ausgeben wenigstens eines zweiten, vom ersten unabhängigen Signals unter Berücksichtigung der gewonnenen Aussage auf. Das Signal kann insbesondere ein Signal sein, welches ein zumindest zeitweiliges bzw. vorübergehendes Unterbrechen einer Steuerung oder einer Regelung der Blutbehandlung, und insbesondere einer hierbei erfolgenden Ultrafiltration, auf den gewonnenen Wert bewirkt.

In einer wiederum weiter bevorzugten Ausführungsform umfasst das Verfahren das Ausgeben eines Signals zum Durchführen einer zumindest zeitweilig konstanten Blutbehandlung und insbesondere einer zumindest zeitweilig konstanten Ultrafiltration. Dieses Signal kann das modifizierte erste, das modifizierte zweite oder ein vom ersten und zweite Signal unabhängiges drittes Signal sein.

Der zumindest zeitweilige Betrieb der Blutbehandlungsvorrichtung mit konstanter Ultrafiltrationsrate und/ oder -leistung bewirkt ein Ausschalten der Ultrafiltrationssteuerung oder -regelung. Dabei können vorzugsweise jedoch Sicherungsmechanismen derart vorgesehen sein, dass Änderungen des Drucks/ Blutdrucks im extrakorporalen Kreislauf oder Abschnitt der Blutbehandlungsvorrichtung, welche aufgrund durch den Anwender geänderter Einstellungen oder bekannter und gewollter Umstände zustande gekommen sind und sehr wohl zu einer Änderung der Ultrafiltrationsrate bzw. -leistung führen sollen, nicht zum Anpassen des Signals führen. Vielmehr soll die Ultrafiltration in solchen Fällen - wie in der Praxis üblich - gestoppt oder vermindert werden. Vorzugsweise kann in einem solchen Fall zusätzlich die Ausgabe eines Warnsignals erfolgen.

Die Aussage über den in einem Abschnitt der Blutbehandlungsvorrichtung oder in einem extrakorporalen Kreislauf herrschenden Druck/ Blutdruck kann in einer wiederum weiter bevorzugten Ausführungsform durch das Messen des Drucks/ Blutdrucks im extrakorporalen Kreislauf, insbesondere in einem venösen Abschnitt hiervon, erfolgen. Dabei kann der gemessene Druck/ Blutdruck als absoluter Wert, als relativer Wert, in einer zeitlichen Ableitung oder auf andere Weise verwendet werden.

Erfindungsgemäß kann ferner, wie in einer wiederum weiter bevorzugten Ausführungsform der Erfindung vorgesehen ist, das relative Blutvolumen zum Gewinnen einer Aussage über das Blutvolumen gemessen werden.

Eine weiter bevorzugte, Ausführungsform des Verfahrens sieht das Ausgeben bzw. Erzeugen eines RBV-Signals unter Berücksichtigung der gewonnenen Aussage zum Steuern oder Regeln der Blutbehandlungsvorrichtung vor.

Mittels des Verfahrens ist es vorteilhaft möglich, bei Auftreten an sich steuerungs- bzw. regelungsrelevanter Abfälle von Werten das Blutvolumen betreffend, wie ein Abfall des RBV-Signals, welche nicht mit einem kritischen Kreislaufzustand einhergehen bzw. Vorboten eines solchen Kreislaufzustandes sind, sondern als Mess-Artefakte bezeichnet werden müssen, auf eine Drosselung der Ultrafiltration zu verzichten. Dies erspart dem Patienten Zeit, die er für die Behandlung aufbringen muss mit allen dem Fachmann bekannten, hiermit verbundenen Vorteilen.

Die erfindungsgemäße Aufgabe wird auch gelöst durch eine Blutbehandlungsvorrichtung gemäß den Merkmalen des Anspruchs 15. Mittels des erfindungsgemäßen Blutvolumenmonitors und auch mittels der erfindungsgemäßen Blutbehandlungsvorrichtung lassen sich alle oben genannten Vorteile ungeschmälert erzielen. Daher wird zur Vermeidung von Wiederholungen an dieser Stelle ausdrücklich auf die obige Diskussion und Darstellung verwiesen.

Die vorliegende Erfindung wird im Folgenden anhand eines in der angehängten Zeichnung dargestellten Ausführungsbeispiels detaillierter erläutert. In der Zeichnung gilt:
- Fig. 1: zeigt ein RBV-Signal und Blutdrücke eines Patienten während einer Dialysebehandlung über der Zeit;
- Fig. 2: zeigt das arterielle und venöse Drucksignal im extrakorporalen Kreislauf; und
- Fig. 3: zeigt schematisch vereinfacht eine erfindungsgemäße Blutbehandlungsvorrichtung.

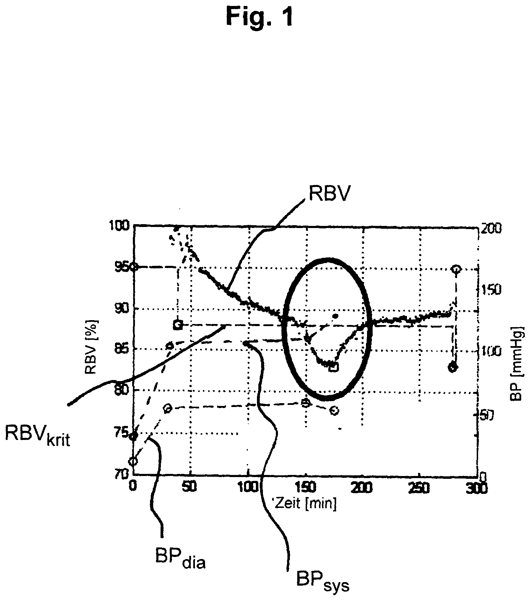

Fig. 1 zeigt sowohl das relative Blutvolumen RBV, in Prozent [%] ausgedrückt, als auch den Blutdruck, in mmHg ausgedrückt, über dem in Minuten skalierten Zeitverlauf der Blutbehandlung eines Patienten. Dabei ist der systolische Blutdruck mit BP_{sys}, der diastolische Blutdruck mit BP_{dia} bezeichnet. In Fig. 1 ist zudem das kritische relative Blutvolumen RBVₖᵢₜ dargestellt. Fällt das RBV-Signal unter diesen kritschen Wert, so wird im vorliegenden Beispiel die Ultrafiltration gesenkt oder ausgesetzt. Es wird angemerkt, dass beim dargestellten Beispiel stets vom Blutdruck die Rede ist. Wie oben erläutert, kann jedoch auch ein anderer Druck gemessen werden. Auch die Messung des RBV steht hier stellvertretend für die Messung eines anderen Blutparameters wie oben stehend ausgeführt ist.

Wie Fig. 1 zeigt, fällt das RBV-Signal im dort dargestellten Beispiel zum Zeitpunkt t = 150 min plötzlich exponentiell um etwa 5 % ab und steigt erst 20 bis 30 Minuten später wieder an. Wie Fig. 1 ebenfalls zu entnehmen ist, geht der starke Abfall des RBV-Signals jedoch nicht auch mit einem Abfall des Blutdrucks einher; sowohl der systolische Blutdruck BP_{sys}, als auch der diastolische Blutdruck BP_{dia} bleiben während des starken RBV-Abfalls im Wesentlichen unverändert. Somit liegt in dem fett umrandeten zeitlichen Bereich jedenfalls kein kritischer Kreislaufzustand vor.

Für den in Fig. 1 dargestellten RBV-Abfall könnten folgende Ursachen verantwortlich sein:
1) Das Refilling vom Interstitium in das Gefäßsystem ändert sich aufgrund Nahrungsaufnahme (bspw. frühstückt der Patient während der Behandlung) oder Änderung der Filtrationsdrücke.
2) Fahraeus-Lindquist-Effekt: Mobilisierung von Blutkompartimenten mit unterschiedlichen Plasmawasser-Konzentrationen. Beispielsweise ist der Hämatokrit HCT in den engen Kapillaren aufgrund der Blockströmung roter Blutkörperchen geringer als in den großen Arterien. Wird ein bislang brachliegendes Kapillarbett zum zirkulierenden Blutvolumen hinzugeschaltet, so strömt plasmawasserreiches Blut in die Kapillare, wodurch der zirkulierende Hämatokrit, welcher über die Ultraschallmessung vom Blutvolumenmonitor BVM erfasst wird, zu einer Verringerung des RBV-Signals führt.

Während einer RBV-basierten Ultrafiltrations-Regelung, bei welcher der RBV-Wert eine kritische Untergrenze RBVₖᵣᵢₜ nicht durchschreiten darf, käme es aufgrund des plötzlichen RBV-Abfalls zu einer signifikanten Verringerung der Ultrafiltration, teilweise sogar zu deren Abschalten für ca. 15-40 Minuten, was negative Auswirkungen auf die Dialysedauer bzw. das Gesamt-Ultrafiltrationsvolumen oder die Reinigungsleistung hat.

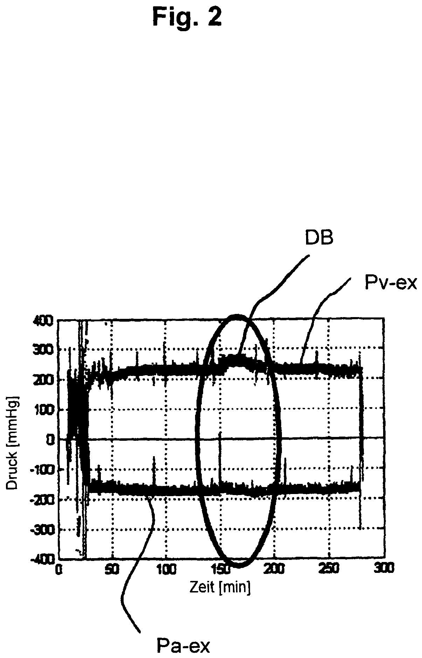

Wie Fig. 2 zu entnehmen ist, in welcher das arterielle Drucksignal Pₐ₋ₑₓ und das venöse Drucksignal Pᵥ₋ₑₓ jeweils des extrakorporalen Kreislaufs dargestellt sind, geht der ab dem Zeitpunkt t = 150 min erfolgende RBV-Abfall - welcher bereits in Fig. 1 gezeigt ist - mit einer Änderung insbesondere im venösen Drucksignal Pᵥ₋ₑₓ einher. Diese Änderung ist in dem fett umrandeten Bereich gut zu erkennen. Sie zeigt sich im venösen Drucksignal als eine *"Druckbeule",* welche in Fig. 2 mit DB bezeichnet ist.

Bei Erkennen einer solchen Druckbeule DB bietet es sich erfindungsgemäß an, beispielsweise das RBV-Signal zu korrigieren. Dies kann in einer erfindungsgemäßen Ausführungsform durch Addition eines druckproportionalen und ggf. patientenspezifischen Wertes zum RBV-Signal erfolgen. Ein weiteres mögliches Vorgehen bei Erkennen einer solchen Druckbeule ist das Ausschalten der Ultrafiltrations-Regelung während der Dauer der Druckbeule. In der Zeit, in welcher die Ultrafiltrations-Regelung ausgeschaltet ist, kann hilfsweise mit einer konstanten Ultrafiltrationsrate weitergefahren werden.

Vorteilhafterweise kann bei jeder Möglichkeit der Korrektur des RBV-Signals oder der vorübergehenden Änderung der Ultrafiltrationsrate sichergestellt werden, dass Druckänderungen, welche z. B. durch Flussänderungen vorhersehbar herbeigeführt werden, nicht zur Artefakterkennung verwendet werden. In diesem Fall würde eine Korrektur des RBV-Signals unterbleiben.

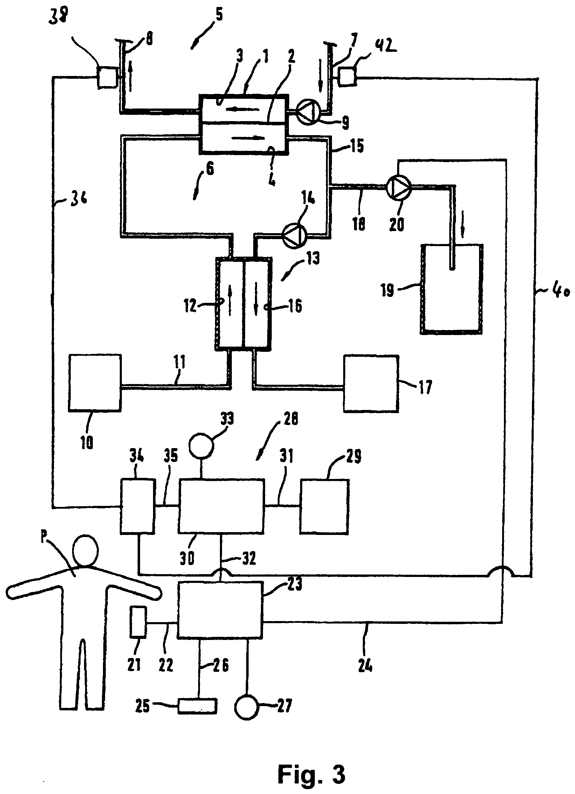

Fig. 3 zeigt die wesentlichen Komponenten einer Hämodiafiltrationsvorrichtung als Beispiel einer erfindungsgemäßen Blutbehandlungsvorrichtung zusammen mit einem erfindungsgemäßen Blutvolumenmonitor in schematischer Darstellung. Der Blutvolumenmonitor kann integraler Bestandteil der Blutbehandlungsvorrichtung (hier: der Hämodiafiltrationsvorrichtung) oder eine separate Einheit sein, die an eine bestehende Blutbehandlungsvorrichtung angeschlossen wird.

Die Hämodiafiltrationsvorrichtung der Fig. 3 umfasst einen Dialysator 1, der durch eine semipermeable Membran 2 in eine Blutkammer 3 und eine Dialysierflüssigkeitskammer 4 unterteilt ist. Die Blutkammer 3 ist in einen extrakorporalen Blutkreislauf 5 geschaltet, die Dialysierflüssigkeitskammer 4 ist in einen Dialysierflüssigkeitskreislauf 6 geschaltet. Von einem Patienten P führt eine Blutzuführleitung 7 zu einem Einlass der Blutkammer 3 des Dialysators 1, und von einem Auslass der Blutkammer 3 führt eine Blutabführleitung 8 zurück zum Patienten P. In die Blutzuführleitung 7 ist eine Blutpumpe 9 geschaltet.

Von einer Dialysierflüssigkeitsquelle 10 führt eine Dialysierflüssigkeitszuführleitung 11 zum Einlass einer ersten Bilanzkammer 12 einer Bilanziereinheit 13 und von deren Auslass zum Einlass der Dialysierflüssigkeitskammer 4 des Dialysators 1.

Eine Dialysierflüssigkeitsabführleitung 15 führt von einem Auslass der Dialysierflüssigkeitskammer 4 zu einer zweiten Bilanzkammer 16 der Bilanziereinheit 13 und von deren Auslass zu einem Abfluss 17. In die Dialysierflüssigkeitsabführleitung 15 ist stromauf der Bilanziereinheit 13 eine Dialysierflüssigkeitspumpe 14 geschaltet.

Stromauf der zweiten Bilanzkammer 16 der Bilanziereinheit 13 zweigt von der Dialysierflüssigkeitsabführleitung 15 eine Ultrafiltrationsleitung 18 ab, die zu einem Abfluss 19 führt, der mit dem Abfluss 17 identisch sein kann. Zum Abziehen des Ultrafiltrats ist in die Ultrafiltratleitung 18 eine Ultrafiltratpumpe 20 geschaltet.

Solange die Ultrafiltratpumpe 20 nicht fördert, verhindert die Bilanziereinheit 13 einen Netto-Austausch von Flüssigkeit zwischen Primär- und Sekundärkreislauf 5, 6. Eine Ultrafiltration findet unter diesen Umständen nicht statt. Die Ultrafiltration wird erst durch das Einschalten der Ultrafiltratpumpe 20 in Gang gesetzt, die aus der Dialysierflüssigkeitskammer 4 des Dialysators 1 gezielt Flüssigkeit entzieht (Ultrafiltration).

Die Blutbehandlungsvorrichtung mit dem Blutvolumenmonitor 28 verfügt über eine Messeinheit 21 zum Bestimmen des Blutvolumens BV des Patienten P während der Blutbehandlung. Die Messeinheit 21 ist über eine Datenleitung 22 mit einer Steuereinheit 23 verbunden, die über eine Datenleitung 24 mit der Ultrafiltratpumpe 20 verbunden ist. Im Allgemeinen ist es für den erfindungsgemäßen Blutvolumenmonitor 28 bzw. zur Durchführung des Verfahrens ausreichend, wenn die Messeinheit 21 relative Angaben über das Blutvolumen des Patienten P zur Verfügung stellt, wie z. B. Angaben über den Wasseranteil im extrakorporalen Blut des Patienten P z. B. in der Leitung 7. Es können auch andere mit dem relativen Blutvolumen korrelierende Messgrössen gemessen werden, wie z. B. eine Hämoglobin- oder Proteinkonzentration. Hierbei kann ein Ultraschallsensor Anwendung finden. Das Vorgehen bei diesen Messungen oder Bestimmungen ist dem Fachmann jeweils bekannt.

Zur Eingabe eines Ultrafiltratgesamtvolumens UFV_{ges}, das während der Gesamt-Behandlungsdauer entzogen werden soll, und zur Eingabe der Behandlungsdauer UFT sowie ggf. weiterer patientenspezifischer Parameter ist eine Eingabeeinheit 25 vorgesehen, die über eine Datenleitung 26 mit der Regeleinheit 23 verbunden ist. Die Regeleinheit 23 stellt in Abhängigkeit von dem mit der Messeinheit 21 gemessenen Blutvolumen BV des Patienten P die Ultrafiltrationsrate UFR(t) der Ultrafiltratpumpe 20 so ein, dass während der vorgegebenen Behandlungsdauer UFT das vorgegebene Ultrafiltratgesamtvolumen UFV_{ges} entzogen wird (Blutvolumenregelung). Hierzu verfügt die Regeleinheit 23 über einen entsprechenden Regelalgorithmus.

Durch Betätigung eines Schalters 27 kann die Regeleinheit 23 deaktiviert und zu einer manuellen Einstellung der Ultrafiltrationsrate übergegangen werden.

Der Blutvolumenmonitor 28 verhindert eine Gefährdung des Patienten P bei einer fehlerhaften Blutvolumenregelung. Der Blutvolumenmonitor 28 umfasst eine Recheneinheit 29 und eine Überwachungseinheit 30, die über eine Datenleitung 31 miteinander kommunizieren. Die Überwachungseinheit 30 ist über eine Datenleitung 32 mit der Regeleinheit 23 verbunden.

Die Recheneinheit 29 des Blutvolumenmonitors 28 kann aus dem vorgegebenen Ultrafiltratgesamtvolumen UFV_{ges} und der vorgegebenen Behandlungsdauer UFT einen oberen Grenzwert für die Ultrafiltrationsrate UFRₗᵢₘ bestimmen.

Der Blutvolumenmonitor 28 weist ferner eine Einrichtung 34 zum Anpassen des RBV-Signals unter Berücksichtigung der Höhe des Blutdrucks im extrakorporalen Kreislauf 5 auf, wobei die Einrichtung 34 mittels einer Datenleitung 35 mit dem Blutvolumenmonitor 28 verbunden ist. Dabei ist die Einrichtung 34 über eine Messleitung 36 zum Messen der Höhe des Blutdrucks mittels eines beliebig (bspw. als Druckdom oder dergleichen) ausgestalteten Drucksensors 38 im extrakorporalen Kreislauf 5 mit dessen venösen Schenkel (Blutabführleitung 8) verbunden. Die ebenfalls dargestellte Messleitung 40, welche ebenfalls einen Drucksensor 42, welcher wie der Drucksensor 38 ausgestaltet sein kann, aufweist, kann zusätzlich oder anstelle der Messleitung 36 vorgesehen sein. Es kann anstelle der Messung im venösen Schenkel - oder zusätzlich zu dieser - auch eine Messung im arteriellen Schenkel (Blutzuführleitung 7) des extrakorporalen Blutkreislaufs 5 vorgesehen sein.

Im Folgenden wird eine mögliche, rein exemplarische Ausführungsweise des Verfahrens beschrieben, welches mittels der in den Figuren diskutierten Vorrichtung ausführbar ist. Das folgende Verfahren kann mehrfach und insbesondere regelmäßig während der Blutbehandlung stattfinden (z. B. einmal pro Minute) und folgende Schritte umfassen:
Das RBV-Signal z. B. der letzten halben Stunde wird linear oder exponentiell approximiert und somit geglättet. Alternativ hierzu kann eine Tiefpassfilterung oder jede andere geeignete Filterung des RBV-Signals erfolgen. Im Folgenden wird das geglättete Signal als Approximation bezeichnet. Das aktuelle RBV-Signal wird mit der für den aktuellen Zeitpunkt intra- oder extrapolierten Approximation verglichen.

Zeigt der Vergleich, dass schneller, starker Abfall des RBV vorliegt (ein Kriterium hierfür könnte z. B. sein, dass die RBV-Abweichung zwischen aktuellem Wert und Approximation größer als z. B. -3 % ist, oder dass ein bestimmter Abfall wie z. B. -3 % in maximal 5 Minuten erfolgt), so werden der arterielle und der venöse Druck im Schlauchsystem betrachtet. Auch hierbei kann ein Vergleich der aktuellen Werte mit entsprechenden Druckapproximationen bspw. über die letzten 5 bis 10 Minuten erfolgen. Dabei wird untersucht, ob schneller, ausgeprägter arterieller und venöser Druckanstieg vorliegt, d. h., es wird untersucht, ob sich der aktuelle Druck in kurzer Zeit (z. B. innerhalb von 5, 10, 20, 30, 40, 50, 60 Sekunden oder 1, 2, 3, 4, oder 5, 6, 7, 8, 9, 10, 11, 12, 13, 14, 15 Minuten oder mehr) von seiner Approximation entfernt. Ein Kriterium für den positiven Fall könnte z. B. sein, dass der arterielle Druck zu seiner Approximation um mehr als 10 mmHg (ebenso sind mehr 5 oder 15 mmHg sowie Zwischenwerte und weitere Werte denkbar) und der venöse Druck zu seiner Approximation um mehr als 5 mmHg (ebenso sind mehr als 3, 4, 6, 7, 8, 9, oder Werte zwischen 10 oder 15 sowie weitere Werte denkbar) abweicht.

Wird dem Kriterium genügt, d. h. entfernt sich der aktuelle Druck von seiner Approximation, so wird ein RBV-Artefakt wegen Lagewechsel (Frühstück, etc.) angenommen und die RBV-Regelung wird ausgeschaltet und eine konstante UF-Rate wird eingestellt. Diese Einstellung kann solange beibehalten werden, bis sich die gleichen oder vergleichbare Effekte in umgekehrter Form oder Richtung abspielen (z. B. ein insbesondere plötzlicher Anstieg des RBV-Signals nach oben bei gleichzeitiger Verminderung der Blutdrücke oder Erreichen des vorherigen RBV-Ausgangswertes vor Einsetzen des Frühstück-Abfalls). Tritt dies ein, so kann die Regelung wieder eingeschaltet werden.

Obgleich in dieser Ausgestaltung der aktuelle RBV-Messpunkt bzw. BP-Messpunkt auf plötzliche, starke Abweichungen hin untersucht wird, ist auch von der Erfindung umfasst, eine Prädiktion des RBV-Signals vorzunehmen, indem man die Approximation auf zukünftige Werte extrapoliert. Dies bietet den Vorteil, dass eine prädiktive Regelung (*"Prädiktion"*) schneller auf manche Ereignisse reagieren kann, was insbesondere dann von Vorteil sein kann, wenn die jeweilige Stellgröße nur langsam ihre Größe oder Gestalt verändert oder träge ist.

Die vorliegende Erfindung schlägt einen Blutvolumenmonitor, konfiguriert zum Ausführen eines Verfahrens zum Betreiben einer Blutbehandlungsvorrichtung für die extrakorporale Blutbehandlung eines Patienten, sowie eine Blutbehandlungsvorrichtung vor.

## Patentansprüche

1. Blutvolumenmonitor, konfiguriert zum Ausführen eines Verfahrens zum Betreiben einer Blutbehandlungsvorrichtung für die extrakorporale Blutbehandlung eines Patienten (P), mit den Schritten
- Gewinnen wenigstens eines Wertes das Blutvolumen (RBV) zu wenigstens einem Zeitpunkt oder dessen Entwicklung über wenigstens zwei Zeitpunkte der Blutbehandlung betreffend;
- Vergleichen des Wertes mit einem vorbestimmten Kriterium für das Blutvolumen (RBV);
- Gewinnen einer Aussage über einen in einem Abschnitt der Blutbehandlungsvorrichtung oder in einem extrakorporalen Kreislauf (5) herrschenden Druck (Pₐ₋ₑₓ, Pᵥ₋ₑₓ) oder dessen Entwicklung;
- Vergleichen der Aussage mit einem vorbestimmten Kriterium für den Druck (Pₐ₋ₑₓ, Pᵥ₋ₑₓ); und
- Überprüfen, bei einem Genügen des Kriteriums für das Blutvolumen (RBV), auf ein Vorliegen einer Koinzidenz, zum Erkennen von Artefakten bei der Blutvolumenmessung, zwischen einem Genügen des Kriteriums für den Druck (Pₐ₋ₑₓ, Pᵥ₋ₑₓ) und einem Genügen des Kriteriums für das Blutvolumen (RBV), wobei Koinzidenz gegeben ist, wenn beiden Kriterien innerhalb einer vorbestimmten Zeitspanne genügt wird.

2. Blutvolumenmonitor (28) nach Anspruch 1, das Verfahren ist **gekennzeichnet durch** die Schritte
Ausgeben wenigstens eines ersten Signals unter Berücksichtigung des gewonnenen Wertes (RBV) oder dessen Entwicklung zum Steuern der Blutbehandlungsvorrichtung, insbesondere zum Steuern einer Ultrafiltrationsrate oder -leistung; und
Anpassen des ersten Signals unter zusätzlicher Berücksichtigung der gewonnenen Aussage (Pₐ₋ₑₓ, Pᵥ₋ₑₓ) über den in einem Abschnitt der Behandlungsvorrichtung oder in einem Abschnitt eines extrakorporalen Kreislaufs (5) herrschenden Druck oder dessen Entwicklung.

3. Blutvolumenmonitor (28) nach Anspruch 2, das Verfahren ist **gekennzeichnet durch** den Schritt
Verändern der Höhe des ausgegebenen ersten Signals zum Steuern der Blutbehandlungsvorrichtung unter Berücksichtigung der gewonnenen Aussage (Pₐ₋ₑₓ, Pᵥ₋ₑₓ) oder deren Entwicklung, insbesondere durch Addition eines druckproportionalen Wertes zum Signal.

4. Blutvolumenmonitor (28) nach einem der Ansprüche 1 bis 3, das Verfahren ist **gekennzeichnet durch** den Schritt
Ausgeben wenigstens eines zweiten Signals unter Berücksichtigung der gewonnenen Aussage (Pₐ₋ₑₓ, Pᵥ₋ₑₓ) oder deren Entwicklung, insbesondere eines Signals zum wenigstens zeitweiligen Unterbrechen einer Steuerung der Blutbehandlung.

5. Blutvolumenmonitor (28) nach einem der vorangegangenen Ansprüche, das Verfahren ist **gekennzeichnet durch** den Schritt
Ausgeben eines Signals zum Durchführen einer zumindest zeitweilig konstanten Blutbehandlung.

6. Blutvolumenmonitor (28) nach einem der vorangegangenen Ansprüche, das Verfahren ist **gekennzeichnet durch** den Schritt
Messen des Drucks, insbesondere des Blutdrucks, im extrakorporalen Kreislauf (5), insbesondere in einem venösen Abschnitt (8) hiervon, zum Gewinnen der Aussage (Pₐ₋ₑₓ, Pᵥ₋ₑₓ) über den extrakorporalen Drucks, insbesondere über den extrakorporalen Blutdruck, oder dessen Entwicklung.

7. Blutvolumenmonitor (28) nach einem der vorangegangenen Ansprüche, das Verfahren ist **gekennzeichnet durch** den Schritt
Messen des relativen Blutvolumens (RBV) zum Gewinnen des Wertes das Blutvolumen betreffend.

8. Blutvolumenmonitor (28) nach einem der Ansprüche 1 bis 7 zum Überwachen einer extrakorporalen Blutbehandlung eines Patienten (P) mittels einer Blutbehandlungsvorrichtung, wobei der Blutvolumenmonitor (28) aufweist:
eine Einrichtung (21) zum Gewinnen wenigstens eines Wertes das Blutvolumen (BV) zu wenigstens einem Zeitpunkt oder dessen Entwicklung über wenigstens zwei Zeitpunkte der Blutbehandlung betreffend; und
eine Einrichtung (36) zum Gewinnen einer Aussage (Pₐ₋ₑₓ, Pᵥ₋ₑₓ) über einen in einem Abschnitt der Blutbehandlungsvorrichtung oder in einem extrakorporalen Kreislauf (5) herrschenden Druck, insbesondere Blutdruck, oder dessen Entwicklung;
**gekennzeichnet durch**
eine Einrichtung zum Überprüfen, bei einem Genügen des Kriteriums für das Blutvolumen (RBV), auf ein Vorliegen einer Koinzidenz, zum Erkennen von Artefakten bei der Blutvolumenmessung, zwischen einem Genügen des Kriteriums für den Druck (Pₐ₋ₑₓ, Pᵥ₋ₑₓ) und einem Genügen des Kriteriums für das Blutvolumen (RBV), wobei Koinzidenz gegeben ist, wenn beiden Kriterien innerhalb einer vorbestimmten Zeitspanne genügt wird..

9. Blutvolumenmonitor (28) nach Anspruch 8, **gekennzeichnet durch**
eine Einrichtung (23) zum Ausgeben wenigstens eines ersten Signals unter Berücksichtigung des gewonnenen Wertes (RBV) zum Steuern der Blutbehandlungsvorrichtung, insbesondere zum Steuern einer Ultrafiltrationsrate oder -leistung; und
eine Einrichtung (34) zum Anpassen des ersten Signals unter zusätzlicher Berücksichtigung der gewonnenen Aussage (Pₐ₋ₑₓ, Pᵥ₋ₑₓ) über den in einem Abschnitt der Behandlungsvorrichtung oder in einem Abschnitt eines extrakorporalen Kreislaufs (5) herrschenden Blutdruck oder dessen Entwicklung.

10. Blutvolumenmonitor (28) nach Anspruch 9, **gekennzeichnet durch**
eine Einrichtung zum Verändern der Höhe des ausgegebenen ersten Signals zum Steuern der Blutbehandlungsvorrichtung unter Berücksichtigung der gewonnenen Aussage (Pₐ₋ₑₓ, Pᵥ₋ₑₓ) oder deren Entwicklung, insbesondere durch Addition eines druckproportionalen Wertes zum Signal.

11. Blutvolumenmonitor (28) nach einem der Ansprüche 9 bis 10, **gekennzeichnet durch**
eine Einrichtung zum Ausgeben wenigstens eines zweiten Signals unter Berücksichtigung der gewonnenen Aussage (Pₐ₋ₑₓ, Pᵥ₋ₑₓ) oder deren Entwicklung, insbesondere eines Signals zum wenigstens zeitweiligen Unterbrechen einer Steuerung der Blutbehandlung.

12. Blutvolumenmonitor (28) nach einem der Ansprüche 8 bis 11, **gekennzeichnet durch**
eine Einrichtung zum Ausgeben eines Signals zum Durchführen einer zumindest zeitweilig konstanten Blutbehandlung.

13. Blutvolumenmonitor (28) nach einem der Ansprüche 8 bis 12, **gekennzeichnet durch**
eine Einrichtung (36) zum Messen des Blutdrucks im extrakorporalen Kreislauf (5) insbesondere in einem venösen Abschnitt (8) hiervon.

14. Blutvolumenmonitor (28) nach einem der Ansprüche 8 bis 13, **gekennzeichnet durch**
eine Einrichtung zum Messen des relativen Blutvolumens (RBV).

15. Blutbehandlungsvorrichtung, insbesondere Dialysevorrichtung, welche geeignet ist zur Aufnahme eines Blutvolumenmonitors (28) nach einem der Ansprüche 9 bis 14.

16. Blutbehandlungsvorrichtung nach Anspruch 15, welche einen Blutvolumenmonitor (28) nach einem der Ansprüche 8 bis 14 aufweist.

17. Blutbehandlungsvorrichtung nach einem der Ansprüche 15 oder 16, welche geeignet ist zur Aufnahme wenigstens eines Blutschlauchsystems (7, 8) für den extrakorporalen Blutkreislauf (5).

18. Blutbehandlungsvorrichtung nach einem der Ansprüche 15 bis 17, welche wenigstens ein Blutschlauchsystem (7, 8) für den extrakorporalen Blutkreislauf (5) aufweist.

19. Blutbehandlungsvorrichtung nach einem der Ansprüche 15 bis 18, welche geeignet ist zur Aufnahme wenigstens eines Hämodialysators (1).

20. Blutbehandlungsvorrichtung nach einem der Ansprüche 15 bis 19, welche einen Hämodialysator (1) aufweist.

## Claims

1. A blood volume monitor configured to execute a method of operating a blood treatment apparatus for the extracorporeal blood treatment of a patient (P), comprising the steps of:
- obtaining at least one value relating to the blood volume (RBV) at at least one point of time or relating to the evolution thereof over at least two points of time of the blood treatment;
- comparing the value with a predetermined criterion for the blood volume (RBV);
- obtaining a statement about a pressure prevailing (Pₐ₋ₑₓ, Pᵥ₋ₑₓ) in a portion of the blood treatment apparatus or in an extracorporeal circuit (5) or about the evolution thereof;
- comparing the statement with a predetermined criterion for the pressure (Pₐ₋ₑₓ, Pᵥ₋ₑₓ); and
- checking, when the blood volume (RBV) criterion is satisfied, the presence of a coincidence between the pressure (Pₐ₋ₑₓ, Pᵥ₋ₑₓ) criterion being satisfied and the blood volume criterion (RBV) being satisfied in order to detect artefacts in the blood volume measurement, wherein a coincidence is given when both criteria are satisfied within a predetermined period of time.

2. The blood volume monitor (28) according to claim 1, wherein the method is **characterized by** the steps of:
- outputting at least one first signal taking into account the obtained value (RBV) or the evolution thereof for controlling the blood treatment apparatus, in particular for controlling an ultrafiltration rate or an ultrafiltration power; and
- adjusting the first signal taking into additional account the obtained statement (Pₐ₋ₑₓ, Pᵥ₋ₑₓ) about the pressure prevailing in a portion of the treatment apparatus or in a portion of an extracorporeal circuit (5) or the evolution thereof.

3. The blood volume monitor (28) according to claim 2, the method being **characterized by** the step of:
- modifying the level of the first signal output to control the blood treatment apparatus taking into account the obtained statement (Pₐ₋ₑₓ, Pᵥ₋ₑₓ) or the evolution thereof, in particular by adding a pressure proportional value to the signal.

4. The blood volume monitor (28) according to anyone of claims 1 to 3, the method being **characterized by** the step of:
- outputting at least one second signal taking into account the obtained statement (Pₐ₋ₑₓ, Pᵥ₋ₑₓ) or the evolution thereof, in particular a signal for at least temporarily interrupting a control of the blood treatment.

5. The blood volume monitor (28) according to anyone of the preceding claims, the method being **characterized by** the step of:
- outputting a signal for performing an at least temporarily constant blood treatment.

6. The blood volume monitor (28) according to anyone of the preceding claims, the method being **characterized by** the step of:
- measuring the pressure, in particular the blood pressure, in the extracorporeal circuit (5), in particular in a venous portion (8) thereof, for obtaining the statement (Pₐ₋ₑₓ, Pᵥ₋ₑₓ) about the extracorporeal pressure, in particular about the extracorporeal blood pressure, or the evolution thereof.

7. The blood volume monitor (28) according to anyone of the preceding claims, the method being **characterized by** the step of:
- measuring the relative blood volume (RBV) to obtain the value relating to the blood volume.

8. The blood volume monitor (28) according to anyone of claims 1 to 7 for monitoring an extracorporeal blood treatment of a patient (P) by means of a blood treatment apparatus, wherein the blood volume monitor (28) comprises:
- a device (21) for obtaining at least one value relating to the blood volume (BV) at at least one point of time, or the evolution thereof over at least two points of time of the treatment; and
- a device (36) for obtaining a statement (Pₐ₋ₑₓ, Pᵥ₋ₑₓ) about a pressure, in particular a blood pressure, prevailing in a portion of the blood treatment apparatus or in an extracorporeal circuit (5), or about the evolution thereof;
**characterized by**
- a device for checking, when the blood volume (RBV) criterion is satisfied, the presence of a coincidence between the pressure (Pₐ₋ₑₓ, Pᵥ₋ₑₓ) criterion being satisfied and the blood volume criterion (RBV) being satisfied in order to detect artefacts in the blood volume measurement, wherein a coincidence is given when both criteria are satisfied within a predetermined period of time.

9. The blood volume monitor (28) according to claim 8, **characterized by**:
- a device (23) for outputting at least one first signal taking into account the obtained value (RBV) to control the blood treatment apparatus, in particular to control an ultrafiltration rate or an ultrafiltration power; and
- a device (34) for adjusting the first signal taking into additional account the obtained statement (Pₐ₋ₑₓ, Pᵥ₋ₑₓ) about the blood pressure prevailing in a portion of the treatment apparatus or in a portion of an extracorporeal circuit (5), or the evolution thereof.

10. The blood volume monitor (28) according to claim 9, **characterized by**:
- a device for modifying the level of the first signal output to control the blood treatment apparatus taking into account the obtained statement (Pₐ₋ₑₓ, Pᵥ₋ₑₓ) or the evolution thereof, in particular by adding a pressure proportional value to the signal.

11. The blood volume monitor (28) according to anyone of claims 9 to 10, **characterized by**:
- a device for outputting at least one second signal taking into account the obtained statement (Pₐ₋ₑₓ, Pᵥ₋ₑₓ) or the evolution thereof, in particular a signal for at least temporarily interrupting a control of the blood treatment.

12. The blood volume monitor (28) according to anyone of claims 8 to 11, **characterized by**:
- a device for outputting a signal for performing an at least temporarily constant blood treatment.

13. The blood volume monitor (28) according to anyone of claims 8 to 12, **characterized by**:
- a device (36) for measuring the blood pressure in the extracorporeal circuit (5), in particular in a venous portion (8) thereof.

14. The blood volume monitor (28) according to anyone of claims 8 to 13, **characterized by**:
- a device for measuring the relative blood volume (RBV).

15. A blood treatment apparatus, in particular a dialysis apparatus, which is suitable for receiving a blood volume monitor (28) according to anyone of claims 9 to 14.

16. The blood treatment apparatus according to claim 15, which comprises a blood volume monitor (28) according to anyone of claims 8 to 14.

17. The blood treatment apparatus according to anyone of claims 15 or 16, which is suitable for receiving at least one blood tubing system (7, 8) for the extracorporeal blood circuit (5).

18. The blood treatment apparatus according to anyone of claims 15 to 17, which comprises at least one blood tubing system (7, 8) for the extracorporeal blood circuit (5).

19. The blood treatment apparatus according to anyone of claims 15 to 18, which is suitable for receiving at least one hemodialyzer (1).

20. The blood treatment apparatus according to anyone of claims 15 to 19, which comprises a hemodialyzer (1).

## Revendications

1. Un moniteur de volume sanguin configuré pour exécuter un procédé de fonctionnement d'un appareil de traitement du sang pour le traitement extracorporel du sang d'un patient (P), comprenant les étapes consistant à:
- obtenir au moins une valeur relative au volume sanguin (RBV) à au moins un moment donné ou relative à son évolution entre au moins deux moments donnés du traitement du sang;
- comparer la valeur à un critère prédéterminé pour le volume sanguin (RBV);
- obtenir une information sur une pression (Pₐ₋ₑₓ, Pᵥ₋ₑₓ) régnant dans une section de l'appareil de traitement du sang ou dans un circuit extracorporel (5), ou sur son évolution;
- comparer l'information à un critère prédéterminé pour la pression (Pₐ₋ₑₓ, Pᵥ₋ₑₓ); et
- vérifier, lorsque le critère du volume sanguin (RBV) est satisfait, la présence d'une coïncidence entre une satisfaction du critère de pression (Pₐ₋ₑₓ, Pᵥ₋ₑₓ) et une satisfaction du critère du volume sanguin (RBV) pour détecter des artefacts lors de la mesure du volume sanguin, une coïncidence existant lorsque les deux critères sont satisfaits dans un laps de temps prédéterminé.

2. Le moniteur de volume sanguin (28) selon la première revendication, où le procédé est **caractérisé par** les étapes consistant à:
- émettre au moins un premier signal tenant compte de la valeur obtenue (RBV) ou de son évolution pour commander l'appareil de traitement du sang, en particulier pour commander un débit ou une puissance d'ultrafiltration; et
- ajuster le premier signal en tenant compte en outre de l'information obtenue (Pₐ₋ₑₓ, Pᵥ₋ₑₓ) sur la pression régnant dans une section de l'appareil de traitement ou dans une section d'un circuit extracorporel (5) ou sur son évolution.

3. Le moniteur de volume sanguin (28) selon la revendication 2, où le procédé est **caractérisé par** l'étape consistant à:
- modifier le niveau du premier signal de sortie pour commander l'appareil de traitement du sang en tenant compte de l'information obtenue (Pₐ₋ₑₓ, Pᵥ₋ₑₓ) ou de son évolution, en particulier en ajoutant au signal une valeur proportionnelle à la pression.

4. Le moniteur de volume sanguin (28) selon l'une quelconque des revendications 1 à 3, où le procédé est **caractérisé par** l'étape consistant à:
- émettre au moins un second signal tenant compte de l'information obtenue (Pₐ₋ₑₓ, Pᵥ₋ₑₓ) ou de son évolution, en particulier un signal pour interrompre au moins de façon temporaire un contrôle du traitement du sang.

5. Le moniteur de volume sanguin (28) selon l'une quelconque des revendications précédentes, où le procédé est **caractérisé par** l'étape consistant à:
- émettre un signal pour effectuer un traitement du sang constant au moins de façon temporaire.

6. Le moniteur de volume sanguin (28) selon l'une quelconque des revendications précédentes, où le procédé est **caractérisé par** l'étape consistant à:
- mesurer la pression, en particulier la pression sanguine, dans le circuit extracorporel (5), en particulier dans une section veineuse (8) de celui-ci, afin d'obtenir l'information (Pₐ₋ₑₓ, Pᵥ₋ₑₓ) sur la pression extracorporelle, en particulier sur la pression sanguine extracorporelle, ou sur son évolution.

7. Le moniteur de volume sanguin (28) selon l'une quelconque des revendications précédentes, où le procédé est **caractérisé par** l'étape consistant à:
- mesurer le volume sanguin relatif (RBV) afin d'obtenir la valeur concernant le volume sanguin.

8. Le moniteur de volume sanguin (28) selon l'une quelconque des revendications 1 à 7 pour surveiller un traitement sanguin extracorporel d'un patient (P) au moyen d'un appareil de traitement du sang, le moniteur de volume sanguin (28) comprenant:
- un dispositif (21) permettant d'obtenir au moins une valeur relative au volume sanguin (BV) à au moins un moment donné ou relative à son évolution entre au moins deux moments donnés du traitement; et
- un dispositif (36) permettant d'obtenir une information (Pₐ₋ₑₓ, Pᵥ₋ₑₓ) sur une pression, en particulier sur une pression sanguine, régnant dans une section de l'appareil de traitement du sang ou dans un circuit extracorporel (5), ou sur son évolution;
**caractérisé par**
- un dispositif permettant de vérifier, lorsque le critère du volume sanguin (RBV) est satisfait, la présence d'une coïncidence entre une satisfaction du critère de pression (Pₐ₋ₑₓ, Pᵥ₋ₑₓ) et une satisfaction du critère du volume sanguin (RBV) pour détecter des artefacts lors de la mesure du volume sanguin, une coïncidence existant lorsque les deux critères sont satisfaits dans un laps de temps prédéterminé.

9. Le moniteur de volume sanguin (28) selon la revendication 8, **caractérisé par**:
- un dispositif (23) permettant d'émettre au moins un premier signal tenant compte de la valeur obtenue (RBV) pour commander l'appareil de traitement du sang, en particulier pour commander un débit ou une puissance d'ultrafiltration; et
- un dispositif (34) permettant d'ajuster le premier signal en tenant compte en outre de l'information obtenue (Pₐ₋ₑₓ, Pᵥ₋ₑₓ) sur la pression régnant dans une section de l'appareil de traitement ou dans une section d'un circuit extracorporel (5) ou sur son évolution.

10. Le moniteur de volume sanguin (28) selon la revendication 9, **caractérisé par**:
- un dispositif permettant de modifier le niveau du premier signal de sortie pour commander l'appareil de traitement du sang en tenant compte de l'information obtenue (Pₐ₋ₑₓ, Pᵥ₋ₑₓ), ou de son évolution, en particulier en ajoutant au signal une valeur proportionnelle à la pression.

11. Le moniteur de volume sanguin (28) selon l'une quelconque des revendications 9 à 10, **caractérisé par**:
- un dispositif permettant d'émettre au moins un second signal tenant compte de l'information obtenue (Pₐ₋ₑₓ, Pᵥ₋ₑₓ), ou de son évolution, en particulier un signal pour interrompre au moins de façon temporaire un contrôle du traitement du sang.

12. Le moniteur de volume sanguin (28) selon l'une quelconque des revendications 8 à 11, **caractérisé par**:
- un dispositif permettant d'émettre un signal pour effectuer un traitement du sang constant au moins de façon temporaire .

13. Le moniteur de volume sanguin (28) selon l'une quelconque des revendications 8 à 12, **caractérisé par**:
- un dispositif (36) permettant de mesurer la pression sanguine dans le circuit extracorporel (5), en particulier dans une section veineuse (8) de celui-ci.

14. Le moniteur de volume sanguin (28) selon l'une quelconque des revendications 8 à 13, **caractérisé par**:
- un dispositif permettant de mesurer le volume sanguin relatif (RBV).

15. Un appareil de traitement du sang, en particulier un appareil de dialyse, qui convient pour recevoir un moniteur de volume sanguin (28) selon l'une quelconque des revendications 9 à 14.

16. L'appareil de traitement du sang selon la revendication 15, qui comprend un moniteur de volume sanguin (28) selon l'une quelconque des revendications 8 à 14.

17. L'appareil de traitement du sang selon l'une quelconque des revendications 15 ou 16, qui convient pour recevoir au moins un système de tubes sanguins (7, 8) pour le circuit sanguin extracorporel (5).

18. L'appareil de traitement du sang selon l'une quelconque des revendications 15 à 17, qui comprend au moins un système de tubes sanguins (7, 8) pour le circuit sanguin extracorporel (5).

19. L'appareil de traitement du sang selon l'une quelconque des revendications 15 à 18, qui convient pour recevoir au moins un hémodialyseur (1).

20. L'appareil de traitement du sang selon l'une quelconque des revendications 15 à 19, qui comprend un hémodialyseur (1).
